(19) Europäisches Patentamt
European Patent Office
Office européen des brevets

(11) Veröffentlichungsnummer: **0 268 053**
**A1**

# (12) EUROPÄISCHE PATENTANMELDUNG

(21) Anmeldenummer: **87114509.0**

(22) Anmeldetag: **05.10.87**

(51) Int. Cl.⁴: **C07D 471/04**, C07D 215/56, A61K 31/435, A61K 31/47, //(C07D471/04,221:00,221:00)

(30) Priorität: **15.10.86 DE 3635062**

(43) Veröffentlichungstag der Anmeldung: **25.05.88 Patentblatt 88/21**

(84) Benannte Vertragsstaaten: **AT BE CH DE ES FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Grohe, Klaus, Dr.**
**Am Wasserturm 10**
**D-5068 Odenthal(DE)**

## (54) Neue Metallsalze von 1-Cyclopropyl-chinoloncarbonsäuren und diese enthaltende Arzneimittel.

(57) Die Erfindung betrifft neue Metallsalze von 1-Cyclopropyl-chinoloncarbonsäuren der Formel I

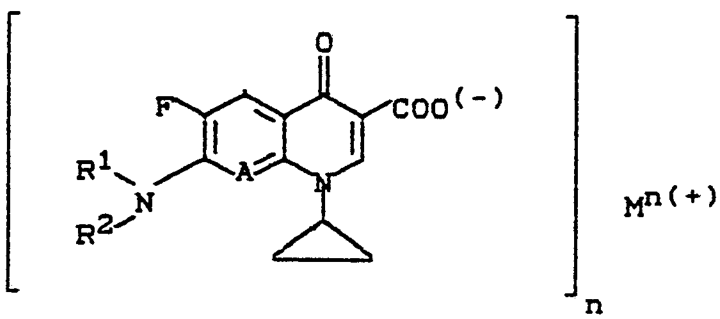

(I)

in welcher
n, M, $R^1$, $R^2$, $R^3$ und A die in der Beschreibung angegebene Bedeutung haben, Verfahren zu ihrer Herstellung sowie ihre Verwendung als Arzneimittel, insbesondere als antibakterielle Mittel in der Human- und Tiermedizin.

EP 0 268 053 A1

Xerox Copy Centre

**Neue Metallsalze von 1-Cyclopropyl-chinoloncarbonsäuren und diese enthaltende Arzneimittel**

Die Erfindung betrifft neue Metallsalze von 1-Cyclopropyl-chinoloncarbonsäuren, Verfahren zu ihrer Herstellung sowie ihre Verwendung als Arzneimittel, insbesondere als antibakterielle Mittel in der Human und Tiermedizin.

Die Erfindung stellt Metallsalze von 1-Cyclopropyl-chinoloncarbonsäuren der Formel I zur Verfügung

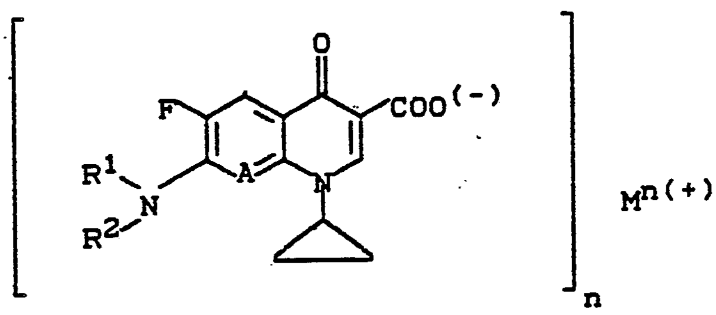

(I)

in welcher n = 1 - 4,
M Silber, Zink, Cer, Kupfer, Mangan, Eisen und Kobalt bedeutet,
$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, $-SO_2-$oder

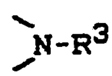

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei
$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy, Alkoxy-, Alkylamino-oder Di-alkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im alipha tischen Teil, einen gegebenenfalls durch Methoxy, Chlor oder Fluor ein-oder zweifach substituierten Phenacylrest, einen Oxoalkylrest mit bis zu 6 Kohlenstoffatomen sowie die Formylgruppe steht,
ferner
A Stickstoff, eine Methin-, Fluormethin-, Chlormethin-, Brommethin-oder Nitromethingruppe bedeutet.

Bevorzugt sind diejenigen Verbindungen der Formel (I), in denen
n I und M Silber darstellt, wobei
A für Stickstoff, die Methin-, Fluormethin-, Chlormethin-und Brommethingruppe steht,
$R^1$ und $R^2$ gemeinsam mit dem Stickstoffatom an das sie gebunden sind, einen 5-oder 6-gliedrigen heterocyclischen Ring bilden können, der zusätzlcih ein Sauerstoffatom oder die Gruppe

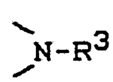

enthalten kann und der gegebenenfalls an den Kohlenstoffatomen ein-bis zweifach durch $C_1$-$C_2$-Alkyl, Phenyl, Hydroxy, Amino, Methylamino, Ethyl amino, Aminomethyl, Methylaminomethyl oder Ethylaminomethyl substituiert sein kann, wobei $R^3$ für Wasserstoff, eine verzweigte oder unverzweigte Alkylgruppe mit 1-3 Kohlenstoffatomen sowie die Formylgruppe steht.

Die erfindungsgemäßen Verbindungen der Formel (I) erhält man, wenn man Alkalisalze von Chinoloncarbonsäuren der Formel (II)

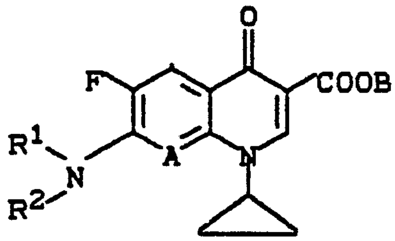

(II)

in der die Reste $R^1$, $R^2$ und A die oben angegebene Bedeutung haben, sowie B für Alkalimetall, bevorzugt Natrium oder Kalium, steht,
mit Metallsalzen wie z.B. Silbernitrat, Cerchlorid, Eisen(II)sulfat, Kupfersulfat oder Zinknitrat umsetzt.

Verwendet man beispielsweise das Natriumsalz der 1-Cyclopropyl-6-fluor-1,4-dihydro-4-oxo-7-(1-piperazinyl)-3-chinolincarbonsäure und Silbernitrat als Ausgangsstoffe, so kann der Reaktionsablauf durch das folgende Formelschema wiedergegeben werden.

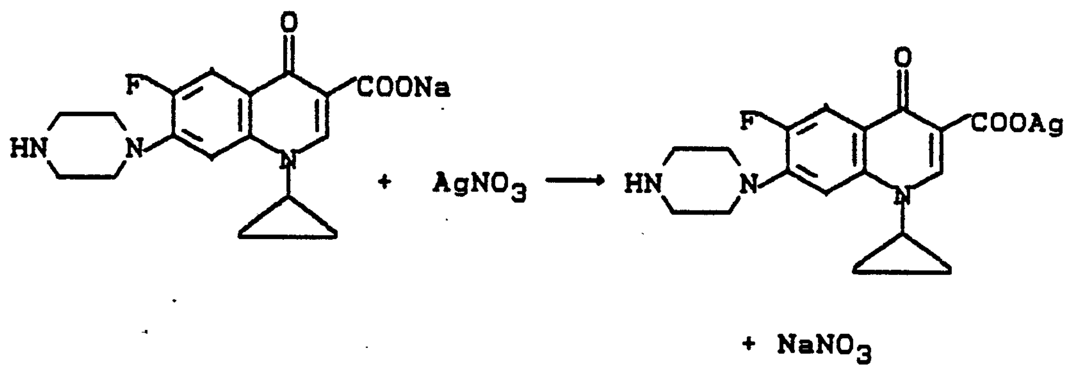


Das in Wasser schwerlösliche Silbersalz fällt aus der Lösung rasch aus und kann abgetrennt werden.

Die Alkalisalze der 1-Cyclopropylchinoloncarbonsäuren können leicht durch Umsetzung der Säuren mit stöchiometrischen Mengen Alkalilauge erhalten werden. Vorteilhafterweise nimmt man etwas weniger Alkalilauge und trennt die überschüssige Chinoloncarbonsäure anschließend durch Filtration ab. Durch Einengen der Lösung erhält man die reinen Alkalisalze.

Die Reaktion wird in einem Temperaturbereich von 0°C bis 100°C, bevorzugt 20°C bis 60°c durchgeführt. Als Verdünnungsmittel wird Wasser verwendet.

Die als Ausgangsstoffe verwendeten 1-Cyclopropyl-chinoloncarbonsäuren sind bekannt.

Als Beispiele seien genannt:

1-Cyclopropyl-6-fluor-1,4-dihydro-4-oxo-7-(1-piperazinyl) -3-chinolincarbonsäure, 1-Cyclopropyl-6-fluor-1,4-dihydro-4-oxo-7-(4-methyl-1-piperazinyl)-3-chinolincarbonsäure, 1-Cyclopropyl-6-fluor-1,4-dihydro-4-oxo-7-(4-ethyl-1-piperazinyl)-3-chinolincarbonsäure, 1-Cyclopropyl-6,8-difluor-1,4-dihydro-4-oxo-7-(1-piperazinyl)-3-chinolincarbonsäure, 1-Cyclopropyl-6,8-difluor-1,4-dihydro-4-oxo-7-(4-methyl-1-piperazinyl)-3-chinolincarbonsäure, 1-Cyclopropyl-6,8-difluor-1,4-dihydro-4-oxo-7-(4-ethyl-1-piperazinyl)-3-chinolincarbonsäure, 1-Cyclopropyl-8-chlor-6-fluor-1,4-dihydro-4-oxo-7-(1-piperazinyl)-3-chinolincarbonsäure, 1-Cyclopropyl-8-chlor-6-fluor-1,4-dihydro-4-oxo-7-(4-methyl-1-piperazinyl)-3-chinolincarbonsäure, 1-Cyclopropyl-8-chlor-6-fluor-1,4-dihydro-4-oxo-7-(4-ethyl-1-piperazinyl)-3-chinolincarbonsäure, 1-Cyclopropyl-6-fluor-1,4-dihydro-4-oxo-7-(3-methyl-1-piperazinyl)-3-chinolincarbonsäure, 1-Cyclopropyl-6,8-difluor-1,4-dihydro-4-oxo-7-(3-methyl-1-piperazinyl)-3-chinolincarbonsäure, 1-Cyclopropyl-8-chlor-6-fluor-1,4-dihydro-4-oxo-7-(3-methyl-1-piperazinyl)-3-chinolincarbonsäure, 1-Cyclopropyl-6-fluor-1,4-dihydro-4-oxo-7-(1-piperazinyl)-1,8-naphthyridin-3-carbonsäure, 1-Cyclopropyl-6-fluor-1,4-dihydro-4-oxo-7-(4-methyl-1-piperazinyl)-1,8-naphthyridin-3-carbonsäure, 1-Cyclopropyl-6-fluor-1,4-dihydro-4-oxo-7-(3-methyl-1-piperazinyl)-1,8-napthyridin-3-carbonsäure, 1-Cyclopropyl-6-fluor-1,4-dihydro-4-oxo-7-morpholinyl-3-chinolincarbonsäure, 1-Cyclopropyl-6-fluor-1,4-dihydro-4-oxo-7-(3-amino-1-pyrrolidinyl)-3-chinolincarbonsäure, 1-Cyclopropyl-6-fluor-1,4-dihdryo-4-oxo-7-(3-methylamino-1-pyrrolidinyl)-3-chinolincarbonsäure, 1-Cyclopropyl-6-fluor-1,4-dihydro-4-oxo-7-(3-ethylaminomethyl-1-pyrrolidinyl)-3-chinolincarbonsäure, 1-Cyclopropyl-6,8-difluor-1,4-dihydro-4-oxo-7-(3-amino-1-pyrrolidinyl)-3-chinolincarbonsäure, 1-Cyclopropyl-6,80-difluor-1,4-dihydro-4-oxo-7-(3-ethylaminomethyl-1-pyrrolidinyl)-3-chinolincarbonsäure, 1-Cyclopropyl-8-chlor-6-fluor-1,4-dihydro-4-oxo-7-(3-ethylamino-1-pyrrolidinyl)-3-chinolincarbonsäure, 1-Cyclopropyl-8-chlor-6-fluor-1,4-dihydro-4-oxo-7-(3-ethylaminomethyl-1-pyrrolidinyl)-3-chinolincarbonsäure, 1-Cyclopropyl-6-fluor-1,4-dihydro-4-oxo-7-(3-ethylaminomethyl-1-pyrrolidinyl-1,8-naphthyridin-3-carbonsäure, 1-Cyclopropyl-6-fluor-1,4-dihydro-4-oxo-7-(3-amino-1-pyrrolidinyl)-1,8-naphthyridin-3-carbonsäure.

Die Metallsalze I weisen eine hohe antibakterielle Wirksamkeit auf. Sie eignen sich besonders für eine Anwendung in der Human-und Tiermedizin. So sind durch Pseudomonas aeruginosa bedingte Infektionen von Wunden und insbesondere Brandwunden nur schwer therapierbar. Neuerdings wurde bekannt, daß 1-Ethyl-chinoloncarbonsäuren und ihre Metallsalze gegen Gentamicin und Silbersulfadiazin resistente Pseudomonas aeruginosa wirksam sind (US 4,522,819).

Die erfindungsgemäßen 1-Cyclopropyl-chinoloncarbonsäuren sind bekanntlich gegen Bakterien wirksamer als die entsprechende 1-Ethylverbindung (Eur. J. Clin. Microbiol. 3, 328 (1984)). Ihre topische Anwendung erscheint daher vorteilhaft.

Die erfindungsgemäßen Verbindungen sind gegen ein sehr breites Spektrum von gramnegativen und grampositiven Bakterien wirksam. Beispielsweise können lokale Erkrankungen behandelt werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bateroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen gebessert oder geheilt werden können, seien beispielsweise genannt:
Infektionskrankheiten beim Menschen wie zum Beispiel Hautinfektionen, postoperative Wundinfektionen, Wundinfektionen, infizierte Verbrennungen und Brandwunden.

Zur vorliegende Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Als bevorzugte pharmazeutische Zubereitungen seien Suspensionen, Pasten, Salben, Gele, Cremes, Lotions und Puder genannt.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure und Talkum oder Gemische dieser Stoffe.

Puder können neben dem oder den Wirkstoffen die üblichen Trägerstoffe enthalten, z.B. Milchzucker, Talkum, Kie selsäure, Calciumsilikat und Polyamidpulver oder Gemische dieser Stoffe.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit-und Sorbitan-Ester, mikrokristalline Cellulose, Bentonit, Aga-Agar und Tragant oder Gemische dieser Stoffe enthalten.

Die therapeutisch wirksamen Verbindungen sollten in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier lokal (Puder, Salbe) angewendet werden. Als geeignete Zubereitungen kommen Gele, Aufgußformulierungen, Emulsionen und Salben in Frage. Zur lokalen Therapie können dermatologische Formulierungen, sowie Puder verwendet werden. Ferner können Gele, Pulver, Puder, Konzentrate, Granulate bei Mensch und Tier angewendet werden. Auch können die

erfindungsgemäßen Verbindungen in andere Trägermaterialien wie zum Beispiel Kunststoffe (Kunststoffketten zur lokalen Therapie), Kollagen oder Knochenzement eingearbeitet werden.

Die folgenden Beispiele erläutern die Erfindung.

Beispiel 1

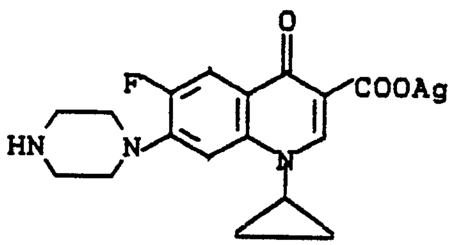

x $3H_2O$

Eine Lösung von 3,5 g (0,01 Mol) des Natriumsalzes der 1-Cyclopropyl-6-fluor-1,4-dihydro-4-oxo-7-(1-Piperazinyl)-3-chinolincarbonsäure in 50 ml Wasser wird mit einer Lösung von 1,75 g Silbernitrat in 25 ml Wasser versetzt. Man rührt 1 Stunde bei Raumtemperatur, saugt den Niederschlag ab, wäscht gut mit Wasser nach und trocknet i. Vak. bei 100°C. Es werden 4,2 g des Silbersalzes der 1-Cyclopropyl-6-fluor-1,4-dihydro-4-oxo-7-(1-piperazinyl)-3-chinoloncarbonsäure als Trihydrat erhalten.

$C_{17}H_{17}AgFN_3O_3$ x $3H_2O$ (491,8)

Ber. C 41,47 H 4,67 N 8,54 F 3,86

Gef. C 41,2 H 4,6 N 8,7 F 3,9

Herstellung des als Ausgangsmaterial verwendeten Natriumsalzes der 1-Cyclopropyl-6-fluor-1,4-dihydro-4-oxo-7-(1-piperazinyl)-3-chinolincarbonsäure:

68 g (0,2 Mol) 1-Cyclopropyl-6-fluor-1,4-dihydro-4-oxo-7-(1-piperazinyl)-3-chinolincarbonsäure werden in 300 ml Wasser suspendiert. Man versetzt mit 7,6 g (0,19 Mol) Ätznatron und rührt 1 Stunde bei Raumtemperatur. Von der überschüssigen Chinolincarbonsäure wird abfiltriert, mit Wasser nachgewaschen und das Filtrat im Vakuum zur Trockne eingedampft. Der Rückstand wird im Vakuum über Calciumchlorid bei 120°C nachgetrocknet. Es werden 73,4 g Natriumsalz vom Zersetzungspunkt >325°C erhalten.

Beispiel 2

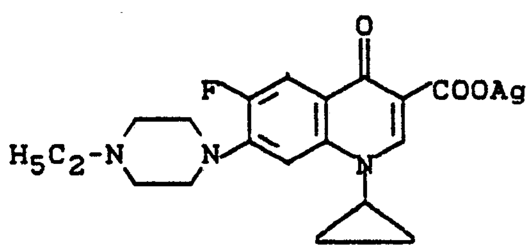

x $2H_2O$

Eine Suspension von 3,6 g (0,01 Mol) 1-Cyclopropyl-6-fluor-1,4-dihydro-4-oxo-7-(4-ethyl-1-piperazinyl)-3-chinolincarbonsäure in 50 ml Wasser wird mit 0,36 g Ätznatron (0,009 Mol) versetzt. Man rührt 30 Minuten bei Raumtemperatur, filtriert und gibt zum Filtrat eine Lösung von 1,75 g Silbernitrat in 25 ml Wasser. Die Suspension wird 1 Stunde gerührt, abgesaugt und der Niederschlag mit Wasser und Ethanol gewaschen. Nach dem Trocknen im Vakuum bei 60 - 80°C über Calciumchlorid werden 4,3 g des Silbersalzes der 1-Cyclopropyl-6-fluor-1,4-dihydro-4-oxo-7-(4-ethyl-1-piperazinyl)-3-chinolincarbonsäure als Dihydrat erhalten.

$C_{19}H_{21}AgFN_3O_3$ x $2H_2O$ (501,8)

Ber. C 45,42 H 4,98 N 8,54 F 8,36

Gef. C 45,3 H 4,9 N 8,7 F 8,2

Beispiel 3

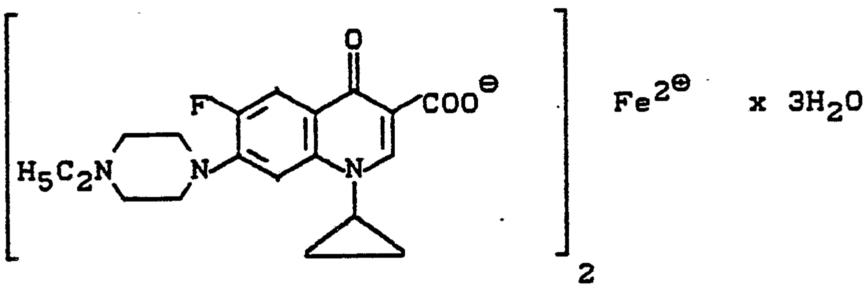

Eine Lösung von 4 g (0,01 Mol) des Kaliumsalzes der 1-Cyclopropyl-6-fluor-1,4-dihydro-4-oxo-7-(4-ethyl-1-piperazinyl)-3-chinolincarbonsäure in 15 ml Wasser wird mit einer Lösung von 1,39 g (0,005 Mol) $FeSO_4 \times 7H_2O$ in 10 ml Wasser versetzt. Man rührt 30 Minuten bei Raumtemperatur, saugt den rotbraunen Niederschlag ab, wäscht gut mit Wasser nach und trocknet im Vakuum bei 100°C. Es werden 3,1 g des Eisen(II)salzes der 1-Cyclopropyl-6-fluor-1,4-dihydro-4-oxo-7-(4-ethyl-1-piperazinyl)-3-chinolincarbonsäure als Trihydrat vom Zersetzungspunkt 323-325°C erhalten.

$C_{38}H_{42}F_2FeN_6O_6 \times 3H_2O$ (825,8)

Ber.: C 55,21 H 5,81 N 10,17 F 4,60 Fe 6,76

Gef.: C 55,0 H 5,8 N 10,2 F 4,6 Fe 6,87

Herstellung des als Ausgangsmaterial verwendeten Kaliumsalzes der 1-Cyclopropyl-6-fluor-1,4-dihydro-4-oxo-7-(4-ethyl-1-piperazinyl)-3-chinolincarbonsäure: 179,5 g (0,5 Mol) 1-Cyclopropyl-6-fluor-1,4-dihydro-4-oxo-7-(4-ethyl-1-piperazinyl)-3-chinolincarbonsäure werden mit 27,5 g Ätzkali in 1200 ml Wasser 1 Stunde bei Raumtemperatur gerührt. Von der überschüssigen Chinolincarbonsäure wird abfiltriert, mit Wasser nachgewaschen und das Filtrat im Vakuum zur Trockne eingedampft. Der Rückstand wird im Vakuum über Calciumchlorid bei 100°C getrocknet. Es werden 170,5 g Kaliumsalz vom Zersetzungspunkt 205°C erhalten.

Beispiel 4

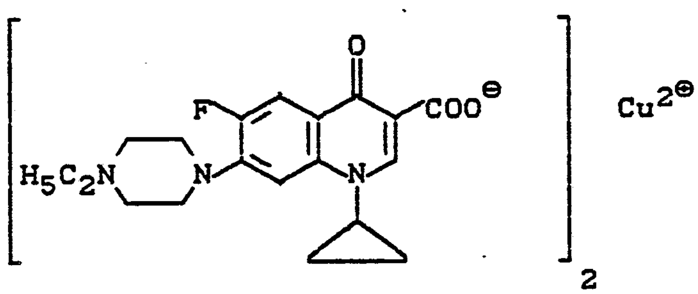

Das Cu(II)-salz der 1-Cyclopropyl-6-fluor-1,4-dihydro-4-oxo-7-(4-ethyl-1-piperazinyl)-3-chinolincarbonsäure wurde analog Beispiel 3 durch Umsetzung von 4 g (0,01 Mol) 1-Cyclopropyl-6-fluor-1,4-dihydro-4-oxo-7-(4-ethyl-1-piperazinyl)-3-chinolincarbonsäure mit 1,25 g (0,005 Mol) $CuSO_4 \times 5H_2O$ erhalten. Ausbeute: 3,5 g Zersetzungspunkt: 272 bis 275°C.

Beispiel 5

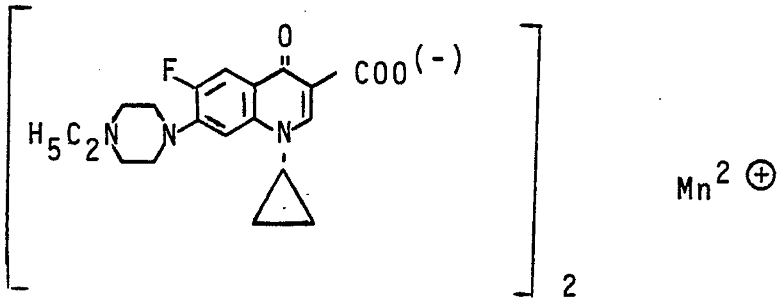

Das Mn (II)-salz der 1-Cyclopropyl-6-fluor-1,4-dihydro-4-oxo-7-(4-ethyl-1-piperazinyl)-3-chinolincarbonsäure wurde analog Beispiel 3 durch Umsetzung von 4 g (0,01 Mol) 1-Cyclopropyl-6-fluor-1,4-dihydro-4-oxo-7-(4-ethyl-1-piperazinyl)-3-chinolincarbonsäure mit 0,85 g (0,005 Mol) $MnSO_4$ x $H_2O$ erhalten.
Ausbeute: 3,6 g
Zersetzungspunkt: 347-350 °C.

Beispiel 6

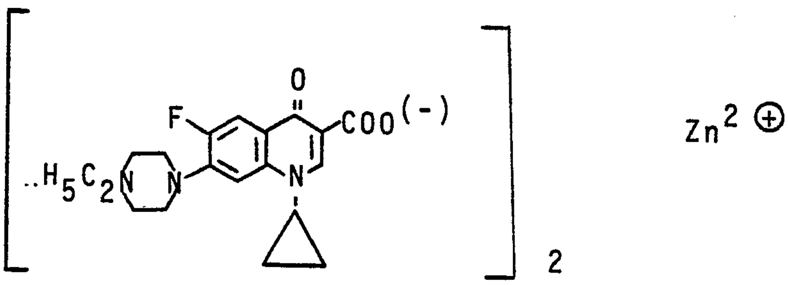

Das Zn (II)-salz der 1-Cyclopropyl-6-fluor-1,4-dihydro-4-oxo-7-(4-ethyl-1-piperazinyl)-3-chinolincarbonsäure wurde analog Beispiel 3 durch Umsetzung von 4 g (0,01 Mol) 1-Cyclopropyl-6-fluor-1,4-dihydro-4-oxo-7-(4-ethyl-1-piperazinyl)-3-chinolincarbonsäure mit 1,44 g (0,005 Mol) $ZnSO_4$ x 7 $H_2O$ erhalten.
Ausbeute: 3,7 g
Zersetzungspunkt: 287-290 °C.

**Ansprüche**

1. Salze von 1-Cyclopropyl-chinoloncarbonsäuren der Formel I

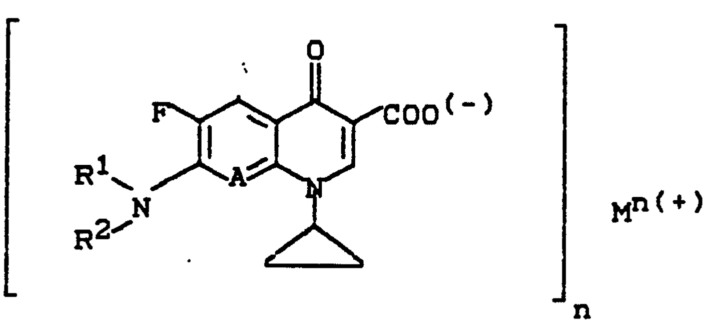

(I)

in welcher
n für 1 bis 4 steht,

M Silber, Zink, Cer, Kupfer, Mangan, Eisen und Kobalt bedeutet,
$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, $-SO_2-$oder

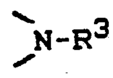

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei
$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Methoxy, Chlor oder Fluor ein-oder zweifach substituierten Phenacylrest, einen Oxoalkylrest mit bis zu 6 Kohlenstoffatomen sowie die Formylgruppe steht, ferner
A Stickstoff, eine Methin-, Fluormethin-, Chlormethin-, Brommethin-oder Nitromethingruppe bedeutet.

2. Salze von 1-Cyclopropyl-chinoloncarbonsäuren der Formel I gemäß Anspruch 1,
in denen
n 1 und M Silber darstellt, wobei
A für Stickstoff, die Methin-, Fluormethin-, Chlormethin-und Brommethingruppe steht,
$R^1$ und $R^2$ gemeinsam mit dem Stickstoffatom an das sie gebunden sind, einen 5-oder 6-gliedrigen heterocyclischen Ring bilden können, der zusätzlich ein Sauerstoffatom oder die Gruppe

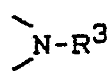

enthalten kann und der gegebenenfalls an den Kohlenstoffatomen ein-bis zweifach durch $C_1$-$C_2$-Alkyl, Phenyl, Hydroxy, Amino, Methylamino, Ethylamino, Aminomethyl, Methylaminomethyl oder Ethylaminomethyl substituiert sein kann, wobei $R^3$ für Wasserstoff, eine verzweigte oder unverzweigte Alkylgruppe mit 1-3 Kohlenstoffatomen sowie die Formylgruppe steht.

3. Salze von 1-Cyclopropyl-chinoloncarbonsäuren der Formel I

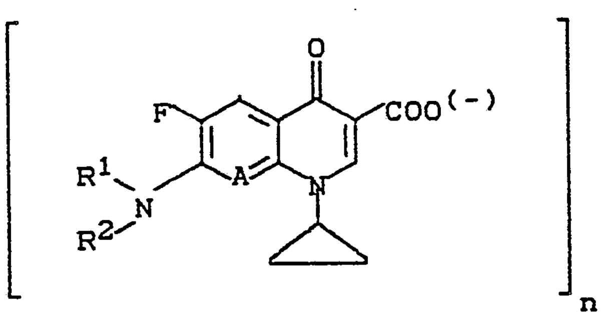

$M^{n(+)}$ (I)

in welcher n = 1 - 4,
M Silber, Zink, Cer, Kupfer, Mangan, Eisen und Kobalt bedeutet,
$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, $-SO_2-$oder

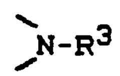

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine ge gebenenfalls im Phenylrest substituierte Phenylalkylgruppe mit bis zu 4 Kohlenstoffatomen im aliphatischen Teil, einen gegebenenfalls durch Methoxy, Chlor oder Fluor eine-oder zweifach substituierten Phenacylrest, einen Oxoalkylrest mit bis zu 6 Kohlenstoffatomen sowie die Formylgruppe steht, ferner

A Stickstoff, eine Methin-, Fluormethin-, Chlormethin-, Brommethin-oder Nitromethingruppe bedeutet,

zur Anwendung in einem Verfahren zur therapeutischen Behandlung des menschlichen oder tierischen Körpers.

4. Verfahren zur Herstellung von Salzen von 1-Cyclopropyl-chinoloncarbonsäuren der Formel I

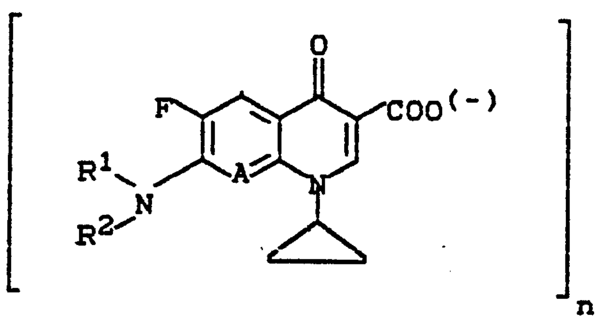

$M^{n(+)}$ (I)

in welcher n = 1 - 4,

M Silber, Zink, Cer, Kupfer, Mangan, Eisen und Kobalt bedeutet,

$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, -$SO_2$-oder

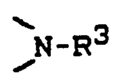

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Methoxy, Chlor oder Fluor ein-oder zweifach substituierten Phenacylrest, einen Oxoalkylrest mit bis zu 6 Kohlenstoffatomen sowie die Formylgruppe steht, ferner

A Stickstoff, eine Methin-, Fluormethin-, Chlormethin-, Brommethin-oder Nitromethingruppe bedeutet, dadurch gekennzeichnet, daß man Alkalisalze von Chinoloncarbonsäuren der Formel (II,

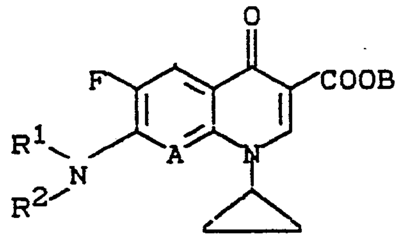

(II)

in der die Reste $R^1$, $R^2$ und A die oben angegebene Bedeutung haben, sowie B für Alkalimetall, bevorzugt Natrium oder Kalium, steht,
mit Metallsalzen aus der Reihe der Silber-, Zink-, Cer-, Kupfer-, Mangan-, Eisen-und Kobaltsalze, z.B. Silbernitrat, Kupfersulfat, Mangansulfat, Cerchlorid oder Zinknitrat umsetzt.

5. Verwendung von 1-Cyclopropyl-chinoloncarbonsäuren der Formel I

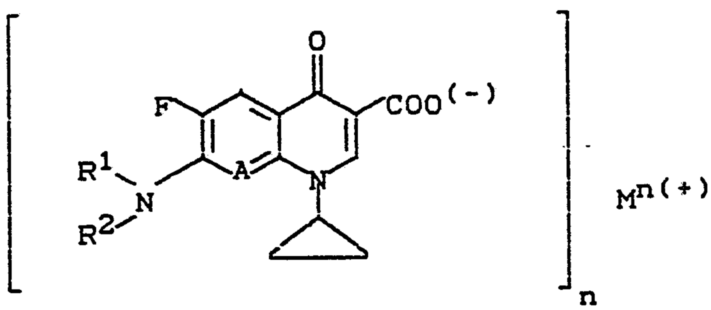

(I)

in welcher n = 1 - 4,
M Silber, Zink, Cer, Kupfer, Mangan, Eisen und Kobalt bedeutet,
$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, $-SO_2$-oder

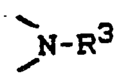

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei
$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Methoxy, Chlor oder Fluor ein-oder zweifach substituierten Phenacylrest, einen Oxoalkylrest mit bis zu 6 Kohlenstoffatomen sowie die Formylgruppe steht, ferner
A Stickstoff, eine Methin-, Fluormethin-, Chlormethin-, Brommethin-oder Nitromethingruppe bedeutet,
zur Herstellung von Arzneimitteln.

6. Arzneimittel enthaltend 1-Cyclopropyl-chinoloncarbonsäuren der Formel I

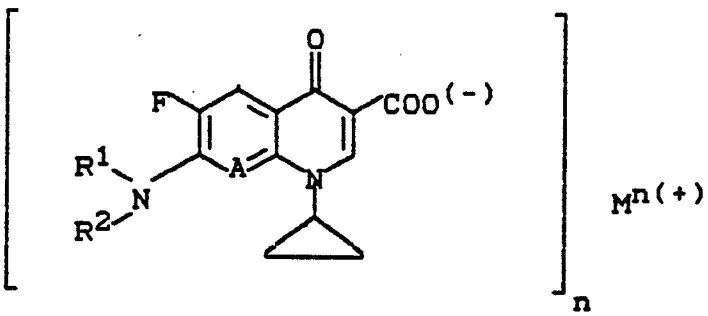

(I)

in welcher n = 1 - 4,

M Silber, Zink, Cer, Kupfer, Mangan, Eisen und Kobalt bedeutet,

$R^1$ und $R^2$ gemeinsam mit dem Stickstoffatom, an das sie gebunden sind, einen 5-oder 6-gliedrigen heterocyclischen Ring bilden, der als Ringlied zusätzlich die Atome oder Gruppen -O-, -S-, -SO-, $-SO_2$-oder

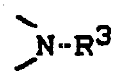

enthalten kann und der gegebenenfalls an den Kohlenstoffatomen ein-bis dreifach, gleich oder verschieden durch $C_1$-$C_4$-Alkyl, gegebenenfalls durch Chlor, Fluor, Brom, Methyl, Phenyl, Hydroxy, Methoxy, Benzyloxy, Nitro oder Piperidino ein-bis dreifach substituiertes Phenyl oder Cyclohexyl, 2-Thienyl, Hydroxy, Alkoxy mit 1 bis 3 Kohlenstoffatomen, Amino, Methylamino, Ethylamino, Aminomethyl, Methylaminomethyl und Ethylaminomethyl substituiert sein kann, wobei

$R^3$ für Wasserstoff, eine verzweigte oder unverzweigte Alkyl-, Alkenyl-oder Alkinylgruppe mit 1 bis 6 Kohlenstoffatomen, die gegebenenfalls durch eine oder zwei Hydroxy-, Alkoxy-, Alkylamino-oder Dialkylaminogruppen mit jeweils 1 bis 3 Kohlenstoffatomen für einen Alkylrest substituiert sein können, die Cyangruppe, die Alkoxycarbonylgruppe mit 1 bis 4 Kohlenstoffatomen im Alkoholteil, eine gegebenenfalls im Phenylrest substituierte Phenylalkylgruppe mit bis zu 4 Kohlenstoffatomen im aliphatischen Teil, einen gegebenenfalls durch Methoxy, Chlor oder Fluor ein-oder zweifach substituierten Phenacylrest, einen Oxoalkylrest mit bis zu 6 Kohlenstoffatomen sowie die Formlygruppe steht, ferner

A Stickstoff, eine Methin-, Fluormethin-, Chlormethin-, Brommethin-oder Nitromethingruppe bedeutet.

Europäisches Patentamt

# EUROPÄISCHER RECHERCHENBERICHT

Nummer der Anmeldung

EP 87 11 4509

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 049 593 (FOX et al.)<br>* Ansprüche 1, 4, 5; Seite 4, Zeile 29 - Seite 5, Zeile 10 * & US - A - 4 522 819 (Kat. D)<br>--- | 1,4-6 | C 07 D 471/04<br>C 07 D 215/56<br>A 61 K 31/435<br>A 61 K 31/47 //<br>(C 07 D 471/04<br>C 07 D 221:00<br>C 07 D 221:00 ) |
| Y | EP-A-0 153 828 (WARNER-LAMBERT CO.)<br>* Ansprüche 1, 2, 5; Seite 9, Zeilen 1-7 *<br>--- | 1,4-6 | |
| P,A | EP-A-0 203 488 (BAYER AG)<br>* Ansprüche 1-4, 8, 11-13 *<br>--- | 1,3,5,6 | |
| A | DE-A-2 843 066 (KYORIN SEIYAKU K.K.)<br>* Ansprüche *<br>----- | 1,5 | |
| | | | **RECHERCHIERTE SACHGEBIETE (Int. Cl.4)** |
| | | | C 07 D 215/00<br>C 07 D 471/00 |

Der vorliegende Recherchenbericht wurde für alle Patentansprüche erstellt

| Recherchenort | Abschlußdatum der Recherche | Prüfer |
|---|---|---|
| BERLIN | 11-01-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)